# EUROPEAN PATENT SPECIFICATION

(11) **EP 2 049 565 B1**
(45) Date of publication and mention of the grant of the patent: **28.01.2015**
(21) Application number: 07787740.5
(22) Date of filing: 19.07.2007
(51) Int. Cl.: C07K 14/14, C07K 19/00, C12N 15/67, C12N 15/62, C12N 15/85

(54) **POSITIVE CYTOMODULINES TO IMPROVE BIOREACTOR PRODUCTIVITY.**
POSITIVE CYTOMODULINE ZUR VERBESSERUNG DER PRODUKTIVITÄT VON BIOREAKTOREN
CYTOMODULINE POSITIVE POUR AMÉLIORER LA PRODUCTIVITÉ D'UN BIORÉACTEUR

(30) Priority: 21.07.2006 US 832096 P
(43) Date of publication of application: 22.04.2009
(73) Proprietor: CENTRE NATIONAL DE LA RECHERCHE SCIENTIFIQUE -CNRS-, 75016 Paris (FR); Institut National De La Recherche Agronomique, 75007 Paris (FR)
(72) Inventor: COSSON, Bertrand, 29670 Taule (FR); LEGAGNEUX, Vincent, 35000 Rennes (FR); PAILLARD, Luc, 35520 Montreuil Le Gast (FR); OSBORNE, Howard Beverley, 35580 Saint Senoux (FR); PONCET, Didier, 91440 Bures sur Yvette (FR); KERYER-BIBENS, Cécile, 44390 Saffre (FR)
(74) Representative: Regimbeau
(86) International application number: PCT/EP2007/057483
(87) International publication number: WO 2008/009727

(56) References cited:
- WO-A-00/53779
- WO-A-02/38738
- WO-A-2005/010038
- APONTE C ET AL: "Expression of two bovine rotavirus non-structural protein (NSP2, NSP3) in the baculovirus system and production of monoclonal antibodies directed against the expressed proteins" ARCHIVES OF VIROLOGY, vol. 133, no. 1-2, 1993, pages 85-95, XP009092613 ISSN: 0304-8608
- RAO ET AL: "Comparative nucleotide and amino acid sequence analysis of the sequence specific RNA-binding rotavirus nonstructural protein NSP3" VIROLOGY, ACADEMIC PRESS,ORLANDO, US, vol. 207, 1995, pages 327-333, XP002987916 ISSN: 0042-6822
- MAUNULA LEENA ET AL: "Frequent reassortments may explain the genetic heterogeneity of rotaviruses: analysis of Finnish rotavirus strains." JOURNAL OF VIROLOGY DEC 2002, vol. 76, no. 23, December 2002 (2002-12), pages 11793-11800, XP002460431 ISSN: 0022-538X & DATABASE UniProt [Online] 1 October 2000 (2000-10-01), "Non-structural protein 3 (Fragment)." retrieved from EBI accession no. UNIPROT:Q9IVH9 Database accession no. Q9IVH9
- PIRON MARIA ET AL: "Identification of the RNA-binding, dimerization, and eIF4GI-binding domains of rotavirus nonstructural protein NSP3" JOURNAL OF VIROLOGY, vol. 73, no. 7, July 1999 (1999-07), pages 5411-5421, XP002460432 ISSN: 0022-538X cited in the application
- VITOUR DAMIEN ET AL: "RoXaN, a novel cellular protein containing TPR, LD, and zinc finger motifs, forms a ternary complex with eukaryotic initiation factor 4G and rotavirus NSP3.", JOURNAL OF VIROLOGY APR 2004 LNKD- PUBMED:15047801, vol. 78, no. 8, April 2004 (2004-04), pages 3851-3862, ISSN: 0022-538X
- VENDE P ET AL: "Efficient translation of rotavirus mRNA requires simultaneous interaction of NSP3 with the eukaryotic translation initiation factor eIF4G and the mRNA 3' end.", JOURNAL OF VIROLOGY AUG 2000 LNKD- PUBMED:10888646, vol. 74, no. 15, August 2000 (2000-08), pages 7064-7071, ISSN: 0022-538X
- KERYER-BIBENS C ET AL: "The rotaviral NSP3 protein stimulates translation of polyadenylated target mRNAs independently of its RNA-binding domain", BIOCHEMICAL AND BIOPHYSICAL RESEARCH COMMUNICATIONS, ACADEMIC PRESS INC. ORLANDO, FL, US, vol. 390, no. 2, 11 December 2009 (2009-12-11), pages 302-306, XP026705129, ISSN: 0006-291X, DOI: 10.1016/J.BBRC.2009.09.115 [retrieved on 2009-10-01]

## Description

### FIELD OF THE INVENTION

The present invention relates to the activation of the protein synthesis by controlling the post-transcriptional steps of the mRNA translation into proteins. The principal industrial application of the invention is the control of the protein production in bioreactors.

### BACKGROUND OF THE INVENTION

Improvements in the production of recombinant proteins using eukaryotic cells are of major importance, in particular for the use of these cells in bioreactors. The production of proteins in bioreactors is expected to increase about 4 fold between 2003 and 2010 ("Optimisation de l'attractivité de la France pour la production biologique" realised in 2004 by the Arthur Doolittle Office for the pharmaceutical companies - LEEM).

The naturally occuring *in vivo* production of proteins in cellular systems is now well known for the skilled person : the pre-messenger RNAs (pre-mRNAs) are transcribed in the nucleus from genomic sequences. They are then matured into mRNAs by the processes of splicing, 3' polyadenylation and 5' capping and subsequently exported to the cytoplasm where they are used by the translation machinery to direct protein synthesis. The cap added to the 5' end of the mRNA is a methylated guanosine triphosphate. In the cytoplasm this modified nucleotide is specifically recognised by the protein complex eIF4F that is required for the recruitment of the small 40S ribosomal subunit to the 5' extremity of the mRNA. Once recruited, the 40S ribosomal subunit moves along the mRNA until a methionine codon (AUG) is encountered. Translation is initiated at this codon after the association of the second ribosomal subunit (60S). Hence, any molecule that increases the efficiency of this chain of events will increase translational efficiency. For a small number of cellular viral mRNAs, the translation initiation uses a mechanism that short cuts the 5' Cap. The conception of the present invention does not use this mode of translational initiation and accordingly it will not be presented here.

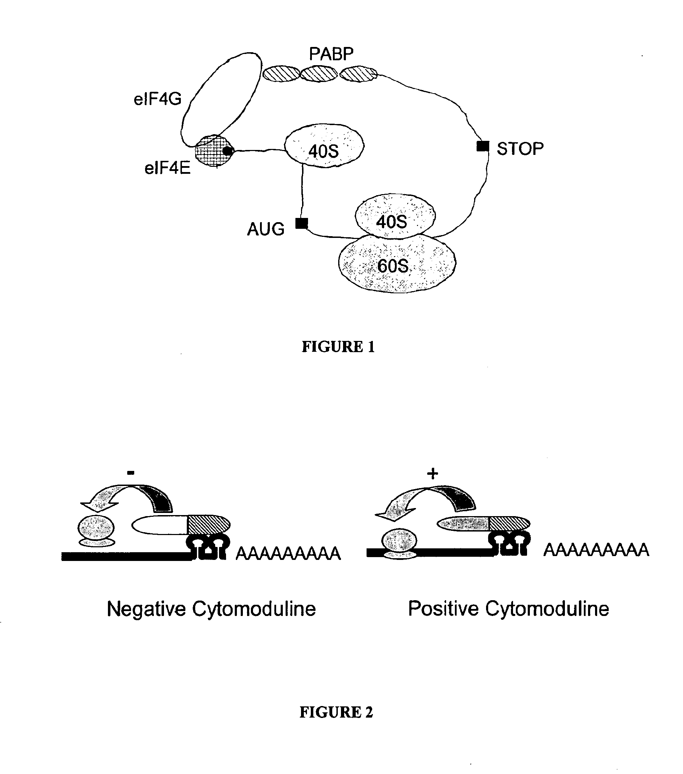

The complex eIF4F contains several translation initiation factors including the proteins eIF4E, that binds to the 5' Cap, and eIF4G (Wickens et al., 2000). This latter factor acts as a platform that allows the assembly of the eIF4F complex. The protein eIF4G can associate with several other proteins, eIF4E as already mentioned, and the poly(A) binding protein (PABP) that binds to the polyadenosine sequence added to the 3' extremity of the mRNA (see Figure 1). The association of the PABP with eIF4G leads to a circularisation of the mRNA, a stabilisation of the eIF4F complex and simulation of cap-dependent translation (Wickens et al., 2000). Any event that destabilises the eIF4F complex causes a decrease in translation. Hence, the loss of the 3' poly(A) tail by an mRNA causes a loss of the PABPs bound to that mRNA which is associated with a decreased translation of the mRNA (Kahvejian et al., 2001; Kahvejian et al., 2005). In contrast, the translation of an mRNA is stimulated when the length of a poly(A) tail increases, an event that causes an increases the number of PABPs associated with the mRNA (Richter, 1999). In this case it would appear that the increased length of the poly(A) tail leads to a greater number of associated PABPs that are available to interact with eIF4G which in turn stimulates translation. It should be noted that the association of the PABP with the poly(A) tail, in addition to the effect on translation described above, also stabilises the mRNA. In cells depleted of PABP the translation is decreased and the degradation of the mRNAs is increased.

Although many aspects of the naturally occurring *in vivo* production of proteins are well known, the step to apply this knowledge in industrial applications, such as to boost the production of recombinant proteins on a large scale, has not been taken in a generalised manner.

### SUMMARY OF THE INVENTION

The inventors have surprisingly discovered that the use of the Non-Structural Protein 3, which is present in rotaviruses, could respond to the industrial requirements of eukaryotic recombinant protein production.

Rotaviruses (Reoviridae family) infect mammalian cells and use the endogenous translation machinery to synthesize the viral proteins (Padilla-Noriega et al., 2002). The viral mRNAs have a 5' cap but no 3' poly(A) tail. Instead, they code for the protein NSP3 (Non-structural protein 3) which, like PABP, binds to a specific sequence at the 3' end of the viral mRNA and to the protein eIF4G (Piron et al., 1998). The interaction between NSP3 and eIF4G occurs via the same domain of eIF4G that interacts with PABP (Piron et al., 1998). In addition, as for the eIF4G-PABP interaction, the complex NSP3-eIF4G stabilises the interaction of eIF4G with eIF4E and consequently the interaction of eIF4E with the cap (Vende et al., 2000). Hence, the protein NSP3 can functionally replace PABP in its role as a translational stimulator. The interaction of NSP3 with RNA and eIF4G occurs via two separate domains (Piron et al., 1999). The crystal structures of each of these domains, associated with the appropriate ligands, have been published (Deo et al., 2002; Groft and Burley, 2002).

The inventors have developed the cytomoduline concept (positive cytomoduline, or cytoboost project) that are hybrid protein molecules, also named herein chimeric polypeptides or proteins, composed in two parts that are functionally distinct, a domain that can specifically interact with an RNA and an effector domain. The RNA-binding domain associates with a mRNA containing the target sequence. This domain acts as a tether which specifically attaches the molecule to the target mRNA. The second domain is the effector domain, which for the cytoboost project, causes a stimulation of the translation of the target mRNA. By varying either the tethering (RNA-binding) or the effector domain it is possible to change, respectively, the RNA target of the cytomoduline or the effect obtained.

As indicated, Cytomodulines are hybrid molecules composed of two domains that are functionally distinct: a tethering and an effector domain (see Figure 2).

The tethering domain of a Cytomoduline binds to an mRNA with sequence specificity. This is necessary for the Cytomoduline to express its function as a regulator of the translation or the stability of targeted mRNAs. Tethering domains can be obtained in several ways. In the present case where cytomodulines are used to increase the production in bioreactors, the Cytomoduline must recognise a recombinant mRNA which gives a greater liberty in the choice of the tethering domain and target sequence. A certain number of RNA-binding proteins have been described in diverse organisms such as eukaryotic cells, virus, bacteriophages and bacteria. Using a tethering domain from a protein normally expressed in eukaryotic cells leads to a problem of Cytomoduline specificity. These molecules would not only bind to the recombinant target mRNA but also to cellular mRNAs that are the endogenous targets of the protein. RNA-binding proteins encoded by the genome of bacteriophages have been chosen.

The effector domain of a positive Cytomoduline must interact with the translation machinery in such a way that the translation is stimulated. To achieve this interaction it should either stabilise or assist in the formation of the ribonucleo-protein complexes of the translation machinery. The present status of research on translational control indicates that regulations exist mainly at the level of the recognition of the 5' Cap or the recruitment of the 40S ribosomal subunit.

The positive Cytomoduline of the invention is based on an effector domain derived from the protein NSP3 that is composed of three domains:
- a N-terminal domain that binds to RNA as a dimer.
- a central domain that appears to be important for the dimerisation.
- a C-terminal domain that binds to eIF4G.

The inventors have thus obtained "positive cytomodulines" ("cytoboost project") or "positive chimeric polypeptides" which are able to activate protein translation. Active molecules (positive cytomodulines) that specifically increase the expression of target proteins in eukaryotic cells are the subject of the present invention. These molecules are effective in bioreactor conditions, for the protein production maximised by the appropriate structural modifications.

An industrial/economic development is proposed in the form of a kit that contains an expression plasmid to receive the gene of interest and a cell line that stably expresses the positive cytomoduline for the application.

The NSP3 protein to be used in the invention may be selected from the NSP3 of the Avian rotavirus A, preferably avian rotavirus AvRV-1, avian rotavirus Ch-1, avian rotavirus PO-13, avian rotavirus RK3, avian rotavirus Ty-1, avian rotavirus Ty-2, or avian rotavirus Ty-3. It may be also a NSP3 protein of the Bovine rotavirus A, such as bovine rotavirus 993/83 or bovine rotavirus RF, caprine rotavirus A, equine rotavirus A, human rotavirus A, preferably human rotavirus (serotype P13 / strain 1845), human rotavirus 1, human rotavirus 2, human rotavirus 4, human rotavirus DG8, human rotavirus G1, G10, G12, G2, G3, G4, G6, G8, or G9, human rotavirus P1B, human rotavirus P3, human rotavirus RMC321, or else untyped human rotaviruses. The NSP3 protein may be further selected from the NSP3 of the lapine rotavirus, such as lapine rotavirus strain BAP (wildtype), lapine rotavirus strain BAP-2, lapine rotavirus strain C-11, lapine rotavirus strain R-2, or rotavirus str. ALA, but also to porcine rotavirus A, preferably porcine rotavirus A strain 134/04-15, rabbit rotavirus, preferably rabbit rotavirus (STRAIN ALABAMA), and rotavirus 5 serotype G2, rotavirus G8, rotavirus subgroup 1, rotavirus subgroup 2, simian rotavirus A/SA11, simian 11 rotavirus (serotype 3 strain SA11-Patton), simian 11 rotavirus (serotype 3 /strain SA11-Ramig), simian rotavirus, simian rotavirus A/SA11-4F, simian rotavirus A/SA11-both, simian rotavirus A/SA11-C14, simian rotavirus A/SA11-FEM, simian rotavirus A/SA11-SEM, swine rotavirus strain S8, or else unclassified Rotavirus A, such as canine rotavirus, canine rotavirus serotype P13 / strain K9, canine rotavirus strain CU-1, lamb rotavirus, rhesus rotavirus, rotavirus GB-503, rotavirus GB-5737, rotavirus RattG1, rotavirus str. 1321, rotavirus strain TUCH, and rotavirus TK159.

Thus, the NSP3 protein may be derived from all the group A retroviruses of the above non-limiting list.

The inventors have determined minimal regions required for an efficient stimulation of the translation positive cytomoduline of the invention ("chimeric polypeptide"), in order to avoid that the NSP3 protein also binds to other proteins. The maximal specificity of the positive cytomoduline has been thus determined.

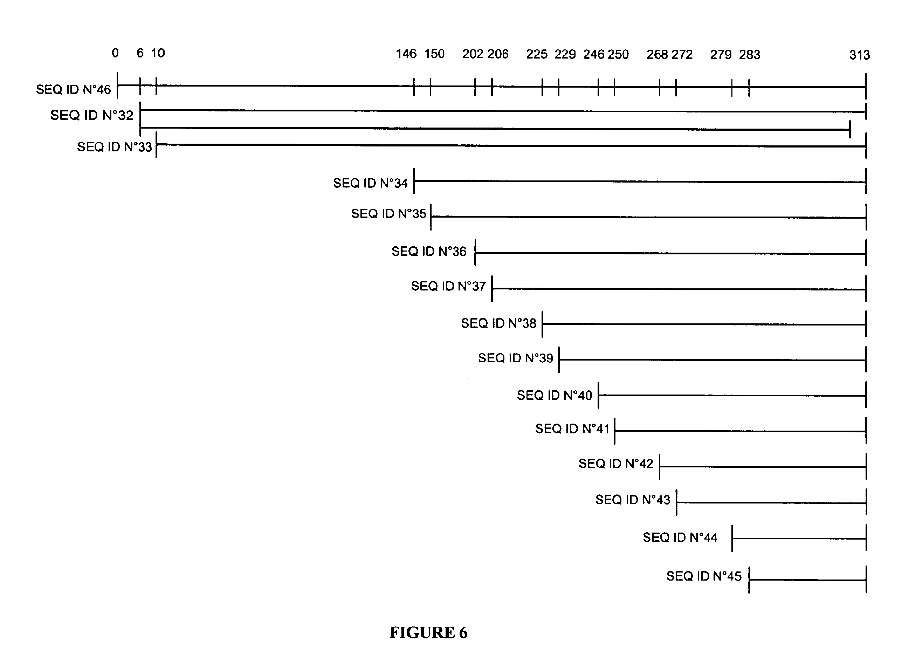

They have shown that some particular fragments of the NSP3 protein of the group A Bovine rotavirus strain RF have the capacity to activate/stimulate protein translation. These peptides are the following: amino acids 10 - 313; 150 - 313; 206 - 313; 229 - 313; 250 - 313; 272 - 313; 283 - 313, and amino acids 6 - 313; 146 - 313; 202 - 313; 225 - 313; 246 - 313; 268 - 313; 279 - 313. The schematic organisation of all these preferred peptides is given in the Figure 6 and the amino-acid sequences thereof is given in the sequence listing, from the sequence SEQ ID N°32 to the sequence SEQ ID N°45. Sequences having at least 70 %, preferably 75 %, 80 %, 85 %, 90 %, 95 %, 96 %, 97 %, 98 % or 99 % of identity with said sequences SEQ ID N°32 to 45 are also within the scope of the invention.

Another fragment of the NSP3 protein of the group A Bovine rotavirus strain RF corresponds to the amino-acids 163 to 313 of the sequence SEQ ID N°46.

Table 3 below indicates identity scores for some rotavirus group A NSP3 proteins.

The minimal region required to increase protein production will be used to make modification aimed at modulating the effect of the cytomoduline. An increased efficiency is important for their use in bioreactors. Site directed mutagenesis of the amino acids that participate either in the interface between NSP3 and eIF4G or in the structuring of that interface should affect the mutual affinity of these two proteins and hence the degree to which translation is stimulated.

The present invention concerns a chimeric polypeptic 6 as defined in claims 1 to 5, a nucleic acid, least cell, protein translation control system and its use as defined in claims 6 to 11.

### DETAILED DESCRIPTION

The terms protein, polypeptide and peptide are used indifferently herein to design an amino acid sequence or their derivatives or analogues containing an amino acid sequence. In the same way, the expressions nucleic acid, nucleotidic acid, polynucleotide, etc.. are used indifferently herein to design a nucleic acid sequence.

By percentage of identity between two amino acid or nucleic acid sequences in the present invention, it is meant a percentage of identical amino acid residues or nucleotides between the two sequences to compare, obtained after the best alignment ; this percentage is purely statistical, and the differences between the two sequences are randomly distributed and all along their length. The best alignment or optimal alignment is the alignment corresponding to the highest percentage of identity between the two sequences to compare, which is calculated such as herein after. The sequence comparisons between two nucleic acid or amino acid sequences are usually performed by comparing these sequences after their optimal alignment, said comparison being performed for one segment or for one "comparison window", to identify and compare local regions of sequence similarity. The optimal alignment of sequences for the comparison can be performed manually or by means of the algorithm of local homology of Smith and Waterman (1981) (Ad. App. Math. 2:482), by means of the algorithm of local homology of Neddleman and Wunsch (1970) (J. Mol. Biol. 48:443), by means of the similarity research method of Pearson and Lipman (1988) (Proc. Natl. Acad. Sci. USA 85:2444), by means of computer softwares using these algorithms (GAP, BESTFIT, FASTA and TFASTA in the Wisconsin Genetics Software Package, Genetics Computer Group, 575 Science Dr., Madison, WI).

The percentage of identity between two amino acid or nucleic acid sequences is determined by comparing these two aligned sequences in an optimal manner with a "comparison window" in which the region of the nucleic acid or amino acid sequence to compare may comprise additions or deletions with regard to the sequence of reference for an optimal alignment between these two sequences. The percentage of identity is calculated by determining the number of positions for which the nucleotide or the amino acid residue is identical between the two sequences, by dividing this number of identical positions by the total number of positions in the "comparison window" and by multiplying the result obtained by 100, to obtain the percentage of identity between these two sequences.

The activating polypeptides as defined above are used to activate in a non specific manner protein translation, preferably in cell systems, although the activating polypeptides may also be used to activate protein translation in acellular systems, such as for example by incubating a cellular mRNA extract constituted of a rabbit reticulocyte lysate according to the conventional method described in UCHIDA et al (2002)

In a second aspect, the invention relates to a chimeric polypeptide capable of specifically activating the translation of a target polynucleotide of interest, which comprises or consists of the Non-Structural Protein 3 (NSP3) of sequence SEQ ID N°46 or a sequence having at least 70 %, preferably 75 %, 80 %, 85 %, 90 %, 95 %, 96 %, 97 %, 98 % or 99 % of identity with SEQ ID N°46 or anyone of the fragments thereof, wherein said NSP3 or anyone of its fragments is fused to a RNA binding protein.

The chimeric polypeptide as defined above is used to activate in a specific manner the translation of a target polynucleotide of interest in the corresponding protein.

Another preferred chimeric polypeptide of the present invention comprises the sequence SEQ ID N°48.

The RNA binding protein of the chimeric polypeptide according to the present invention may be one of the proteins as disclosed in the Table 1 below:

**Table 1:**

| RNA binding protein | Reference |
|---|---|
| MS2 | J. COLLER et al., Genes Dev. 1998 Oct 15;12(20):3226-35; WITHERELL et al. Prog Nucleic Acid Res Mol Biol. 1991;40:185-220 |
| N | DE GREGORIO et al., EMBO J. 1999 Sep 1;18(17):4865-74; TAN & FRANKEL, Biochemistry. 1994 Dec 6;33(48):14579-85 |
| IRP | DE GREGORIO et al., RNA. 2001 Jan;7(1):106-13 ; HENZE & KUHN, Proc Natl Acad Sci U S A. 1996 Aug 6;93(16):8175-82. Review |
| U1A | BRODSKY & SILVER, RNA. 2000 Dec;6(12):1737-49 |

Thus, in one further embodiment, the invention is directed to a chimeric polypeptide according to the present invention, wherein the RNA binding protein is selected from the group consisting of MS2CP, N, IRP and U1A. Preferably, the RNA binding protein is MS2CP containing mutations or deletions that retain the ability to dimerise but which prevent aggregation (Peabody and Ely, 1992; Le Cuyer et al., 1995) or genetic fusions of two MS2CP coding sequences (Peabody and Lim, 1996) containing or not the aforementioned mutations of deletions.

Other RNA binding proteins not listed in Table 1 are also within the scope of the invention.

Advantageously, the selected RNA binding protein is localised at the N-terminal extremity of the chimeric polypeptide, although it may be also localised at the C-terminal extremity of said chimeric polypeptide.

In the chimeric polypeptide, the RNA binding protein may be fused directly to the effector domain, which means:
(i) when the RNA binding protein is localised at the N-terminal part of the chimeric polypeptide, the last amino-acid localised in C-terminal position of the RNA binding protein is chemically linked, preferably by a normal peptidic linkage, to the amino-acid localised at the N-ter position of the effector domain; or
(ii) when the RNA binding protein is localised at the C-terminal part of the chimeric polypeptide, the amino-acid localised in C-terminal position of the effector domain is chemically linked, preferably by a normal peptidic linkage, to the amino-acid localised at the N-ter position of the RNA binding protein.

In another embodiment, the RNA binding protein and the effector domain are not directly linked with each other, but are on the contrary separated in the chimeric polypeptide by a spacer amino-acid sequence or "linker", which is preferably hydrophobic. The spacer amino-acid sequence has a size which is sufficient to constitute a flexible region of the amino-acid molecule, allowing a relative mobility of the RNA binding protein.

The spacer sequence is between 3 and 50 amino-acids long, preferably between 5 and 30 amino-acids long, and most preferably between 5 and 20 amino-acids long.

Preferably, when the spacer sequence or spacer peptide is hydrophobic, said spacer peptide makes it easier for the polypeptide to penetrate the cell membrane. In such a case, said spacer peptide contains a majority of hydrophobic amino-acids such as the amino-acids valine, leucine or isoleucine.

In this embodiment, the spacer peptide comprises in its sequence preferably at least 50 % of hydrophobic amino-acids, more preferably at least 60 % of hydrophobic amino-acids, most preferably at least 80 % of hydrophobic amino-acids.

In a particular embodiment, the spacer peptide consists in a poly(alanine) amino-acid chain, comprising from 3 to 50, preferably from 5 to 30, more preferably from 5 to 20 and most preferably from 5 to 10 alanine amino-acids.

In another embodiment, the spacer peptide consists of 3 to 50 amino acids and most preferably 5 to 10 amino-acids the sequence of which does not modify the function of the cytomoduline. For example, said spacer peptide may consist in the sequence SEQ ID N°50.

In still another embodiment, the spacer peptide is a tag allowing the detection or the purification of the chimeric polypeptide present in a sample. For example, the spacer peptide may consist in the "HA TAG" peptide of sequence SEQ ID N°31.

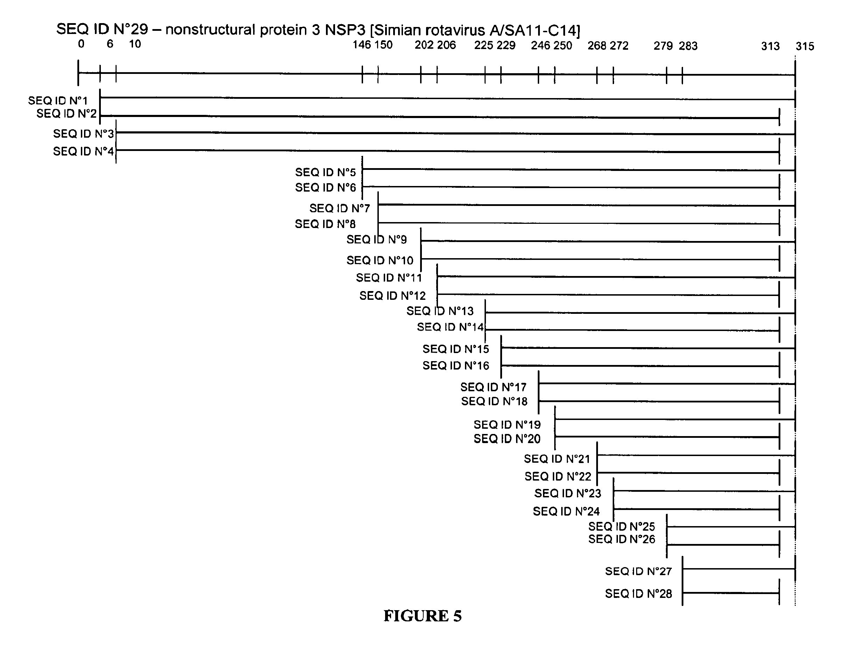

In a more preferred embodiment, the sequences having at least 70 % of identity with anyone of sequences SEQ ID N°32 to SEQ ID N°46 originate from anyone of the following rotaviruses : bovine rotavirus A, caprine rotavirus A, equine rotavirus A, human rotavirus A, lapine rotavirus, porcine rotavirus A, rabbit rotavirus, rotavirus 5 serotype G2, rotavirus G8, rotavirus subgroup 1, rotavirus subgroup 2, simian rotavirus A/SA11, simian rotavirus A/SA11-C14 and swine rotavirus strain S8. Preferably, it originates from the simian rotavirus A/SA11-C14 (accession number: AAL58537) and the corresponding preferred fragments are disclosed above and in the Figure 5.

Preferably, the NSP3 protein to be used in the invention is selected from the NSP3 of the Avian rotavirus A, preferably avian rotavirus AvRV-1, avian rotavirus Ch-1, avian rotavirus PO-13, avian rotavirus RK3, avian rotavirus Ty-1, avian rotavirus Ty-2, or avian rotavirus Ty-3. It may be also a NSP3 protein of the Bovine rotavirus A, such as bovine rotavirus 993/83, caprine rotavirus A, equine rotavirus A, human rotavirus A, preferably human rotavirus (serotype P13 / strain 1845), human rotavirus 1, human rotavirus 2, human rotavirus 4, human rotavirus DG8, human rotavirus G1, G10, G12, G2, G3, G4, G6, G8, or G9, human rotavirus P1B, human rotavirus P3, human rotavirus RMC321, or else untyped human rotaviruses. The NSP3 protein may be further selected from the NSP3 of the lapine rotavirus, such as lapine rotavirus strain BAP (wildtype), lapine rotavirus strain BAP-2, lapine rotavirus strain C-11, lapine rotavirus strain R-2, or rotavirus str. ALA, but also to porcine rotavirus A, preferably porcine rotavirus A strain 134/04-15, rabbit rotavirus, preferably rabbit rotavirus (STRAIN ALABAMA), and rotavirus 5 serotype G2, rotavirus G8, rotavirus subgroup 1, rotavirus subgroup 2, simian rotavirus A/SA11, simian 11 rotavirus (serotype 3 / strain SA11-Patton), simian 11 rotavirus (serotype 3 / strain SA11-Ramig), simian rotavirus, simian rotavirus A/SA11-4F, simian rotavirus A/SA11-both, simian rotavirus A/SA11-C14, simian rotavirus A/SA11-FEM, simian rotavirus A/SA11-SEM, swine rotavirus strain S8, or else unclassified Rotavirus A, such as canine rotavirus, canine rotavirus serotype P13 / strain K9, canine rotavirus strain CU-1, lamb rotavirus, rhesus rotavirus, rotavirus GB-503, rotavirus GB-5737, rotavirus RattG1, rotavirus str. I321, rotavirus strain TUCH, and rotavirus TK159.

Preferably, the chimeric polypeptide according to the present invention further comprises a transport signal which allows the chimeric polypeptide to cross the plasma membrane of the target cells. Transport signals are well known from the skilled person. It may be for example spacer peptides as described above.

In a third aspect, the invention relates to a nucleic acid comprising or consisting of a polynucleotide encoding a polypeptide according to the present invention. Preferably, said nucleic acid further comprises an inducible regulatory polynucleotide for the control of the polynucleotide encoding the polypeptide according to the present invention.

In a fourth aspect, the invention relates to a nucleic acid comprising or consisting of a polynucleotide encoding a chimeric polypeptide according to the present invention. In an advantageous embodiment, said nucleic acid comprises or consists of the sequence SEQ ID N°49. Preferably, said nucleic acid further comprises an inducible regulatory polynucleotide for the control of the polynucleotide encoding the chimeric polypeptide according to the present invention.

The invention also relates to a nucleic acid comprising a polynucleotide encoding a chimeric polypeptide as defined herein and which also comprises a regulatory polynucleotide sensitive to the direct or indirect action of an inducer signal or agent, also disclosed herein as an inducible regulatory polynucleotide.

In practice, the control of the translation of certain predetermined target proteins by a positive chimeric polypeptide according to the invention implies that at particular moments of a cell culture, for example in a bioreactor, the target protein(s) is(are) not produced, whereas at another moments the production is on contrary searched for.

Preferred inducible regulation systems are listed in the Table 2 below:

**Table 2:**

| NAME | PROMOTER | INDUCER | BIBLIOGRAPHICAL OR COMMERCIAL REFERENCES |
|---|---|---|---|
| pMSG | MMTV-LTR «(mouse mammary tumor virus ») | Dexamethasone | Amersham Phannacia |
| pOPRSVI/MCS | RSV-LTR ("Rous sarcoma virus") | IPTG | Stratagene |
| pTet-Splice | Tet | Tetracycline | Life Technologies |
| pTRE | hCMV-1 | Tetracycline or doxycycline | Clontech |
| pRev-TRE | hCMV-1 | Tertracycline or doxycycline | Clontech |
| ²pRetro-On pRetro-Off | hCMV-1 | Tetracycline or doxycycline | Clontech |
| pIND series | ΔHSP («Heat shock protein ») | Ecdysone | Invitrogen |
| pPOP | mPGK/lacO (phosphoglycérate kinase) | IPTG | G.N. Hannan, S.A. Lehnert, E.S. MacAvoy, P.A. Jennings and P.L. Molloy, An engineered PGK promoter and lac operator-repressor system for the regulation of gene expression in mammalian cells. Gene 130 (1993), pp. 233-239. |
| pEF-LAC | hEF-1α/lacO | IPTG | Edamatsu, H., Kaziro, Y., and Itoh, H. (1997) Inducible high level expression vector for mammalian cells, pEF-LAC carrying human elongation factor 1 α promoter and lac operator. Gene 187, 289-294. |
| pBPVMT1 | mMT-I (metallothionein I) | Cd⁺⁺, Zn⁺⁺, PMA⁺⁺ | Pavlakis, G.N., and Hamer, D.H. (1983) Regulation of a metallothionein-growth hormone hybrid gene in bovine papilloma virus. Proc. Natl. Acad. Sci. USA 80, 397-401 |
| pMT | hMT-II (metallothionein II) | Cd⁺⁺, Zn++, PMA⁺⁺ | Friedman, J.S., Cofer, C.L., Anderson, C.L., Kushner, J.A., Gray, P.P., Chapman, G.E., Stuart, M.C., Lazarus, L., Shine, J., and Kushner, P.J. (1989) High expression in mammalian cells without amplification. Bio/Technology 7, 359-362 |
| pMT302 | hMT-IIA (mutant) | Cd⁺⁺, Zn⁺⁺, PMA⁺⁺ | Makarov, S.S., Jonat, C., and Haskill, S. (1994) Hyperinducible human metallothionein promoter with a low level basal activity. Nucleic Acids Res. 22 1504-1505 |
| pIPF | hIFN-α, (interferon α) | Virus | Mori, T., Yamamoto, K., Ohta, T., Sakamoto, C., Sato, M., Koide, K., Murakami, T., Fujii, M., Fukuda, S., and Kurimoto, M. (1994) A high level and regulatable production system for recombinant glycoproteins using a human interferon - α promoter-based expression vector. Gene 144-289-293 |
| pGRE5 | 5XGRE/Ad2MLP "(glucocorticoid reponse element/adenovirus major late promoter") | Dexamethasone | Mader, S., and White, J.H. (1993) A steroid-inducible promoter for the controlled overexpression of cloned genes in eukaryotic cells. |
| GRE5 | "high affinity glucocorticoid reponse element (GRE)/Adenovirus 2MLP" | Dexamethasone | S.Mader and White, A steroid-inducible promoter for the controlled overexpression of clonec genes in eukaryotic cells. *Proc. Natl. Acad. Sci. USA* 90 (1993), pp.5603-5607 0(12) 5603-7 |
| pRDB | "DRE/MMTV (dioxin reponse element)" | TTCD' | .A.De Benedetti and R.E. Rhoads, A novel BK virus-based episomal vector for expression of foreign genes in mammalian cells. Nucleic Acids Res. 19 (1991), pp.1925-1931. |

In a further aspect, the invention relates to a vector comprising a nucleic acid according to the present invention.

In a further aspect, the invention relates to a host cell which stably or transiently expresses a chimeric polypeptide, comprising:
- a nucleic acid according to the present invention, which comprises or consists of a polynucleotide encoding the chimeric polypeptide, or a vector comprising said nucleic acid.

Preferably, the host cell further comprises:
- an expression plasmid encoding a target polynucleotide of interest.

By the term vector or plasmid in the sense of the present invention, it is meant a DNA or RNA, circular or linear molecule, which is indifferently in the form of a single or double strand.

A recombinant vector according the invention is preferably an expression vector. It may be a vector of bacterial or viral origin.

In any case, the nucleic acid encoding the polypeptide or the chimeric polypeptide of the invention is under the control of one or several sequences containing regulating signals of its expression in cells.

The choice of expression control sequence and expression vectors will depend upon the choice of host. A wide variety of expression host/vector combinations may be employed. The preferred bacterial vectors according for the invention may be for example the vectors pBR322 (ATCC N 37017) or else the vectors such as pAA223-3 (Pharmacia, Uppsala, Sweden) and pGEM1 (PromegaBiotech, Madison, W1, USA). Other commercialised vectors may be cited, such as the vectors pQE70, qQE60, Pqe9 (QUIAGEN), psiX174, pBluescript SA, pNH8A, pMH16A, pMH18A, pMH46A, pWLNEO, pSV2CAT, pOG44,pXTI and pSG (Stratagene).

Useful expression vectors for eukaryotic hosts, include, for example, vectors comprising expression control sequences from SV40, bovine papilloma virus, adenovirus and cytomegalovirus. Useful expression vectors for bacterial hosts include known bacterial plasmids, such as plasmids from E. coli, including col E1, pCR1, pBR322, pMB9 and their derivatives, wider host range plasmids, such as M13 and filamentous single stranded DNA phages. Preferred E. coli vectors include pL vectors containing the lambda phage pL promoter (U.S. Pat. No. 4,874,702), pET vectors containing the T7 polymerase promoter (Studier et al., Methods in Enzymology 185: 60-89, 1990) and the pSP72 vector (Kaelin et al., supra). Useful expression vectors for yeast cells include the 2.mu. plasmid and derivatives thereof. Useful vectors for insect cells include pVL 941. Useful expression vectors for plant cells include, but are not limited to, cauliflower mosaic virus (CaMV) and tobacco mosaic virus (TMV). In addition, any of a wide variety of expression control sequences may be used in these vectors. Such useful expression control sequences include the expression control sequences associated with structural genes of the foregoing expression vectors. Examples of useful expression control sequences include, for example, the early and late promoters of SV40 or adenovirus, the lac system, the trp system, the TAC or TRC system, the major operator and promoter regions of phage lambda, for example pL, the control regions of fd coat protein, the promoter for 3-phosphoglycerate kinase or other glycolytic enzymes, the promoters of acid phosphatase, e.g., Pho5, the promoters of the yeast .alpha.-mating system and other sequences known to control the expression of genes or prokaryotic or eukaryotic cells and their viruses, and various combinations thereof.

Any suitable host may be used to produce in quantity the polypeptides of the present invention described herein, including bacteria, fungi (including yeasts), plant, insect, mammal, or other appropriate animal cells or cell lines, as well as transgenic animals or plants. More particularly, these hosts may include well known eukaryotic and prokaryotic hosts, such as strains of E.coli, Pseudomonas, Bacillus, Streptomyces, fungi, yeast, insect cells such as Spodoptera Frugiperda (SF9), and animal cells such as Chinese hamster ovary (CHO), mouse NS/O cells, African green monkey cells such as COS1, COS 7, BSC 1, BSC 40, and BMT 10, and human cells such as human embryonic kidney cells (HEK 293), as well as plant cells in tissue culture.

It should of course be understood that not all vectors and expression control sequences will function equally well to express the polypeptide or the polypeptidic complex of the present invention. Neither will all hosts function equally well with the same expression system. However, one of skill in the art may make a selection among these vectors, expression control systems and hosts without undue experimentation.

Any of the methods known in the art for the insertion of polynucleotide sequences into a vector may be used. See, for example, Sambrook et al., Molecular Cloning: A Laboratory Manual, Cold Spring Harbor Laboratory, Cold Spring Harbor, N.Y. (1989) and Ausubel et al., Current Protocols in Molecular Biology, J. Wiley & Sons, NY (1992), both of which are incorporated herein by reference. Conventional vectors consist of appropriate transcriptional/translational control signals operatively linked to the polynucleotide sequence for producing the polypeptide or the polypeptidic complex of the present invention. Promoters/enhancers may also be used to control expression of polypeptide or the polypeptidic complex of the present invention. Promoter activation may be tissue specific or inducible by a metabolic product or administered substance. Such promoters/enhancers include, but are not limited to, the native E2F promoter, the cytomegalovirus immediate-early promoter/enhancer (Karasuyama et al., J. Exp. Med., 169: 13 (1989)); the human beta-actin promoter (Gunning et al., Proc. Nat. Acad. Sci. USA, 84: 4831 (1987); the glucocorticoid-inducible promoter present in the mouse mammary tumor virus long terminal repeat (MMTV LTR) (Klessig et al., Mol. Cell. Biol., 4: 1354 (1984)); the long terminal repeat sequences of Moloney murine leukemia virus (MuLV LTR) (Weiss et al., RNA Tumor Viruses, Cold Spring Harbor Laboratory, Cold Spring Harbor, N.Y. (1985)); the SV40 early region promoter (Bernoist and Chambon, Nature, 290:304 (1981)); the promoter of the Rous sarcoma virus (RSV) (Yamamoto et al., Cell, 22:787 (1980)); the herpes simplex virus (HSV) thymidine kinase promoter (Wagner et al., Proc. Nat. Acad. Sci. USA, 78: 1441 (1981)); the adenovirus promoter (Yamada et al., Proc. Nat. Acad. Sci. USA, 82: 3567 (1985)).

Expression vectors compatible with mammalian host cells include, for example, plasmids; avian, murine and human retroviral vectors; adenovirus vectors; herpes viral vectors; and non-replicative pox viruses. In particular, replication-defective recombinant viruses can be generated in packaging cell lines that produce only replication-defective viruses. See Current Protocols in Molecular Biology: Sections 9.10-9.14 (Ausubel et al., eds.), Greene Publishing Associcates, 1989.

Specific viral vectors for use in gene transfer systems are now well established. See for example: Madzak et al., J. Gen. Virol., 73: 1533-36 (1992: papovavirus SV40); Berkner et al., Curr. Top. Microbiol. Immunol., 158: 39-61 (1992: adenovirus); Moss et al., Curr. Top. Microbiol. Immunol., 158: 25-38 (1992: vaccinia virus); Muzyczka, Curr. Top. Microbiol. Immunol., 158: 97-123 (1992: adeno-associated virus); Margulskee, Curr. Top. Microbiol. Immunol., 158: 67-93 (1992: herpes simplex virus (HSV) and Epstein-Barr virus (HBV)); Miller, Curr. Top. Microbiol. Immunol., 158: 1-24 (1992: retrovirus); Brandyopadhyay et al., Mol. Cell. Biol., 4: 749-754 (1984: retrovirus); Miller et al., Nature, 357: 455-450 (1992: retrovirus); Anderson, Science, 256: 808-813 (1992: retrovirus), all of which are incorporated herein by reference.
Nucleic acids encoding the polypeptide of the invention can be introduced into eukaryotic cells growing in culture in vitro by conventional transfection techniques (e.g., calcium phosphate precipitation, DEAE-dextran transfection, electroporation, lipofection, etc.). Nucleic acid can also be transferred into cells in vivo, for example by application of a delivery mechanism suitable for introduction of nucleic acid into cells in vivo, such as retroviral vectors (see e.g., Ferry, N et al. (1991) Proc. Natl. Acad. Sci. USA 88:8377-8381; and Kay, M. A. et al. (1992) Human Gene Therapy 3:641-647), adenoviral vectors (see e.g., Rosenfeld, M. A. (1992) Cell 68:143-155; and Herz, J. and Gerard, R. D. (1993) Proc. Natl. Acad. Sci. USA 90:2812-2816), receptor-mediated DNA uptake (see e.g., Wu, G. and Wu, C. H. (1988) J. Biol. Chem. 263:14621; Wilson et al. (1992) J. Biol. Chem. 267:963-967; and U.S. Pat. No. 5,166,320), direct injection of DNA (see e.g., Acsadi et al. (1991) Nature 332:815-818; and Wolff et al. (1990) Science 247:1465-1468) or particle bombardment (see e.g., Cheng, L. et al. (1993) Proc. Natl. Acad. Sci. USA 90:4455-4459; and Zelenin, A. V. et al. (1993) FEBS Letters 315:29-32). Appropriate culture media to be used for cultivating recombinant host cells and production of recombinant proteins are well known from the man skilled in the art.

In a further aspect, the invention relates to a protein translation control system of a target polynucleotide of interest comprising a chimeric polypeptide according the present invention, or a nucleic acid according to the present invention, which comprises or consists of a polynucleotide encoding the chimeric polypeptide, or a vector comprising said nucleic acid, or a host cell according to the present invention. Preferably, the protein translation control system further comprises an expression plasmid encoding a target polynucleotide of interest.

In a further aspect, the invention relates to a method for controlling *in vitro* the translation of a target polynucleotide of interest, which comprises the following steps:
(a) introducing in a host cell the target polynucleotide of interest;
(b) cultivating the recombinant host cell obtained at step (a) in an appropriate culture medium, thus allowing said host cell to stably express said target polynucleotide of interest; and
(c) adding to the culture medium a chimeric polypeptide according to the present invention in order to activate the expression of said target polynucleotide of interest.

In a further aspect, the invention relates to a method for controlling *in vitro* the translation of a target polynucleotide of interest, which comprises the following steps:
(a) introducing in a host cell the target polynucleotide of interest as well as a nucleic acid according to the present invention, which comprises or consists of a polynucleotide encoding the chimeric polypeptide, or a vector comprising said nucleic acid;
(b) cultivating the recombinant host cell obtained at step (a) in an appropriate culture medium, thus allowing said host cell to stably express said target polynucleotide of interest; and
(c) adding to the culture medium an agent capable to interact with the inducible regulatory polynucleotide, thus allowing to regulate the expression of the nucleic acid encoding the chimeric polypeptide.

In a further aspect, the invention relates to a kit for controlling the translation of a target polynucleotide of interest, comprising a chimeric polypeptide according the present invention, or a nucleic acid according to the present invention, which comprises or consists of a polynucleotide encoding the chimeric polypeptide, or a vector comprising said nucleic acid.

Preferably, the kit further comprises a recombinant vector comprising the target polynucleotide of interest.

In a further aspect, the invention relates to a kit for controlling the translation of a target polynucleotide of interest, comprising a host cell according to the present invention. Preferably, said kit further comprises a recombinant vector comprising the target polynucleotide of interest.

Kits typically comprise two or more components necessary for performing said detection or for performing a diagnostic assay. Components may be compounds, reagents, containers and/or equipment.

In a further aspect, the invention relates to the use of a protein translation control system according to the present invention or of a kit according to the present invention for activating the translation of a target polynucleotide of interest in a bioreactor comprising host cells in culture.

The invention also relates to a method for the production of a polypeptide according to the invention or a chimeric polypeptide according to the invention, comprising the following steps:
a) inserting a nucleic acid encoding the polypeptide in an appropriate expression vector,
b) cultivating, in an appropriate culture medium, a host cell previously transformed or transfected with the recombinant vector obtained at step (a),
c) recovering the conditioned culture medium or lyse the host cell, for example by sonication or by osmotic change,
d) separating and purifying, from the culture medium, or else from the cell lysates obtained at step (c), said polypeptide,
e) optionally, characterising the produced recombinant polypeptide.

The polypeptides of the invention may be characterised by fixation on an immunoaffinity chromatography column on which antibodies directed to the polypeptide have been first immobilised.

In another aspect, a polypeptide according to the invention or a chimeric polypeptide according to the invention, may be purified by migration in a series of chromatography columns, according to well known methods, described in AUSUBEL et al. (1989, Current Protocols in Molecular Biology, Green Publishing Associates and Wiley Interscience N. Y.). Said polypeptide may also be prepared by the classical techniques of chemical synthesis, in homogeneous solution or in solid phase. As an illustrative example, a polypeptide according to the invention or a chimeric polypeptide according to the invention, may be prepared by the technique in homogeneous solution disclosed by HOUBEN WEIL (1974, In methode der Organischen Chemie, E.Wunshed., volume 15-1 et15-li, Thieme, Stuttgart) or else by the technique of synthesis in solid phase disclosed by MERRIFIELD (1965a, Nature, vol. 207 (996) : 522-523; 1965b Science, vol. 150 (693) : 178-185).

Lastly, the invention relates to a pharmaceutical composition comprising a chimeric polypeptide according to the present invention.

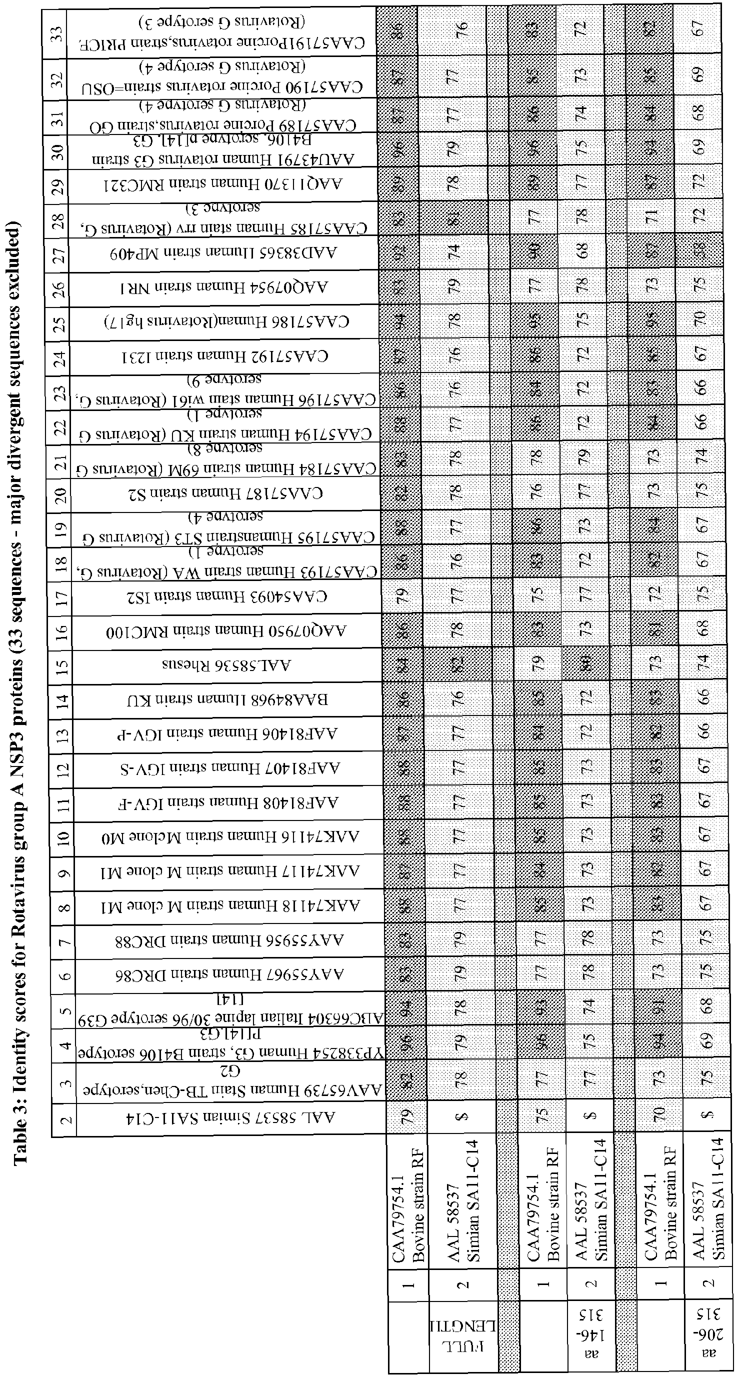

The invention is further embodied in the following examples and figures.

### FIGURES

**Figure 1****. Cap-dependent translation.**
   The factor eIF4G (open ellipse) acts a platform on which the factor eIF4E (cross hatched), bound to the cap (black dot) at the 5' end of the mRNA, and the PABP (diagonal hatched), associated with the 3' poly(A) tail, can bind. The 40S and 60S ribosomal sub-units (grey ellipses), the initiation (AUG) and termination (STOP) codons are indicated (black squares).
**Figure 2****. General structure of a Cytomoduline.**
   Cytomodulines with both positive (Cytoboost) or negative (anti-viral) effects are depicted on the left or right respectively by ellipses. The tethering (cross hatched) and effecter (white) domains are distinct. In principle, the same tethering domain can be used for both positive and negative Cytomodulines. This possibility is used to test the effecter domains.

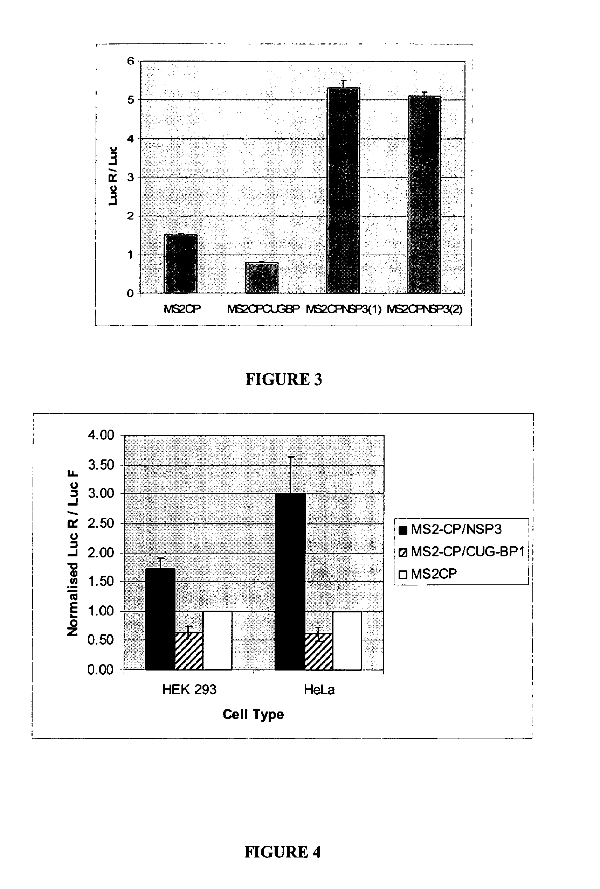

**Figure 3****. Stimulation of translation by the prototype Cytomoduline.**
   Hela cells, were co-transfected with a plasmid encoding a Cytomoduline and a second plasmid from which two reporter mRNAs are transcribed that encode individually the Renilla and Firefly Luciferases. The identity of the Cytomoduline expressed is indicated below the graph. In the reporter plasmid the reading frame encoding the Renilla Luciferase is followed by the MS2 stem loop sequence recognised by the MS2CP protein present in the Cytomodulines. The activity of the Firefly Luciferase allows a normalisation with respect to the transfection efficiency. The Cytomoduline MS2CP/CUG-BP1, prototype of an inhibitory Cytomoduline is used here as a negative control. The two bars for the MS2CPNSP3 Cytomoduline correspond to two separate experiments. In these experiments the positive Cytomoduline was encoded by a genetic in-frame fusion of two MS2-CP sequences followed by amino acids 163 to 313 of bovine NSP3 RF stain (see sequence SEQ ID N°48). The Luciferase activity is expressed as the ratio of Renilla to Firefly activities. The values given are the average ± standard deviation for triplicate assays in an individual transfection experiments.
**Figure 4****. Stimulation of translation by the prototype Cytomoduline: comparison of the effect in HeLa and HEK 293 cells.**
   Hela or HEK 293 cells, as indicated below the figure, were co-transfected with a plasmid encoding a Cytomoduline and a second plasmid from which two reporter mRNAs are transcribed that encode individually the Renilla and Firefly Luciferases. The identity of the Cytomoduline expressed is indicated on the right of the graph. The plasmids encoding the reporter mRNA and cytomoduline are the same as those described in the legend to figure 3. The Luciferase activity is expressed as the ratio of Renilla to Firefly activities (Luc R / Luc F) relative to this ratio observed for the Cytomoduline containing MS2CP alone. The values given are the average ± standard deviation of data obtained in three separate experiments (n= 3).
**Figure 5****. Schematic organisation of the peptides derived from the NSP3 protein of the group A rotavirus strain Simian SA11**
**Figure 6****. Schematic organisation of the peptides derived from the NSP3 protein of the group A bovine rotavirus strain RF**

### EXAMPLES

The inventors have obtained a positive Cytomoduline. This positive Cytomoduline is composed of a RNA-binding or tethering domain containing the phage protein MS2CP and an effector domain derived from the viral protein NSP3. In a first demonstration the effect of this Cytomoduline on the expression of a reporter protein was studied in HeLa cells, which is a standard established human cell line derived from a Human Negroid cervix epitheloid carcinoma. The effect of the positive Cytomoduline on protein expression in these cells was compared with that caused by the presence of a previously described negative Cytomoduline derived from the same tethering protein (MS2-CP) fused with the human protein CUG-BP1 (Peptide protein translation inhibitor and the use thereof for protein translational control, WO2005010038 - 2005-02-03). As a further control the effect of expressing the tethering protein alone (fused with a C-terminal HA tag) on reporter protein expression was also determined. The mRNA encoding the reporter protein (Renila Luciferase) was transcribed from a bidirectonal CMV promoter within a transfected plasmid. In this mRNA the 3' untranslated regions that followed the Renilla Luciferase coding region, contained multiple binding sites for the tethering protein. The gene encoding the Firefly Luciferase was also expressed from this same bidirectional CMV promoter. This mRNA was devoid of binding sites for the tethering protein and hence allowed normalisation of the transfection efficiency. Accordingly, the data is expressed as the ratio of Renila to Firefly luciferase activities (Luc R/Luc F).

In figure 3 are shown the data obtained for two separate transfections of HeLa cells with the positive Cytomoduline (MS2CP/NSP3), for cells transfected with the negative Cytomoduline MS2-CP/CUG-BP1 and for cells transfected with the HA tagged form of MS2CP. Taking the normalised expression of Renilla Luciferase in the presence of HA tagged MS2CP as a base line, the expression on Renilla Luciferase decreased in the presence of the negative Cytomoduline as would be expected. In contrast, the expression of Renilla Luciferase was strongly enhanced in cells that also expressed the positive Cytomoduline (MS2CP/NSP3).

To ensure that this stimulatory effect of the positive Cytomoduline was not restricted to HeLa cells, these measurements were repeated using HEK 293 cells. HEK 293 cells also have an epithelial morphology and originated from a Human Embryo Kidney. The results for this second demonstration are given in figure 4 where the data for the HEK 293 cells is compared with that from HeLa cells. The data for the HeLa cells shown in figure 4 are distinct from those shown in figure 3 and result from new experiments. For both cell types, three independent transfections were made of each the effector plasmids. Again, the expected decrease of Renilla Luciferase activity was observed when the negative Cytomoduline was expressed in either cell type. Furthermore, the positive Cytomoduline also stimulated the expression of Renilla Luciferase in HEK 293 cells showing that the effect is not restricted to a single cell type.

### REFERENCES

Barnes, L. M., Bentley, C. M., and Dickson, A. J. (2003) Stability of protein production from recombinant mammalian cells. Biotechnol Bioeng. 81, 631-639.
Butler, M. (2005) Animal cell cultures: recent achievements and perspectives in the production of biopharmaceuticals. Appl Microbiol Biotechnol. 68, 283-291.
Deo, R. C., Groft, C. M., Rajashankar, K. R., and Burley, S. K. (2002) Recognition of the rotavirus mRNA 3' consensus by an asymmetric NSP3 homodimer. Cell. 108, 71-81.
Groft, C. M., and Burley, S. K. (2002) Recognition of eIF4G by rotavirus NSP3 reveals a basis for mRNA circularization. Mol Cell. 9, 1273-1283.
Kahvejian, A., Roy, G., and Sonenberg, N. (2001) The mRNA closed-loop model: the function of PABP and PABP-interacting proteins in mRNA translation. Cold Spring Harb Symp Quant Biol. 66, 293-300.
Kahvejian, A., Svitkin, Y. V., Sukarieh, R., M'Boutchou, M. N., and Sonenberg, N. (2005) Mammalian poly(A)-binding protein is a eukaryotic translation initiation factor, which acts via multiple mechanisms. Genes Dev. 19, 104-113.
LeCuyer, K.A., Behlen, L.S. and Uhlenbeck, O.C. (1995) Mutants of the bacteriophage MS2 coat protein that alter its cooperative binding to RNA. Biochemistry 34, 10600-10606.
Padilla-Noriega, L., Paniagua, O., and Guzman-Leon, S. (2002) Rotavirus protein NSP3 shuts off host cell protein synthesis. Virology. 298, 1-7.
Peabody, D.S. and Ely, K.R. (1992) Control of translational repression by protein-protein interactions. Nucleic Acids Res. 20, 1649-1655.
Peabody, D.S. and Lim, F. (1996) Complementation of RNA binding site mutations in MS2 coat protein heterodimers. Nucleic Acids Res. 24, 2352-2359.
Piron, M., Vende, P., Cohen, J., and Poncet, D. (1998) Rotavirus RNA-binding protein NSP3 interacts with eIF4GI and evicts the poly(A) binding protein from eIF4F. Embo J. 17, 5811-5821.
Piron, M., Delaunay, T., Grosclaude, J., and Poncet, D. (1999) Identification of the RNA-binding, dimerization, and eIF4GI-binding domains of rotavirus nonstructural protein NSP3. J Virol. 73, 5411-5421.
Richter, J. D. (1999) Cytoplasmic polyadenylation in development and beyond. Microbiol Mol Biol Rev. 63, 446-456.
Uchida et al, J. Biol. Chem., Dec. 2002; 277:50286-50292.
Vende, P., Piron, M., Castagne, N., and Poncet, D. (2000) Efficient translation of rotavirus mRNA requires simultaneous interaction of NSP3 with the eukaryotic translation initiation factor eIF4G and the mRNA 3' end. J Virol. 74, 7064-7071.
Wickens, M., Goodwin, E., Kimble, J., Strickland, S., and Hentze, M. (2000). Translational control in developmental decisions. In Translational control of gene expression, (J. Hershey, M. Mathews, and N. Sonenberg, eds.) pp. 295-370, Cold Spring Harbor Laboratory Press, Cold Spring Harbor NY.

## Claims

1. A chimeric polypeptide capable of specifically activating the translation of a target polynucleotide of interest, which comprises a polypeptide consisting in an amino-acid fragment of the NSP3 protein of sequence SEQ ID N°46, said amino-acid fragment being selected from the group consisting of:
- the fragment 150-313 which has the sequence SEQ ID N°35,
- any sequence having at least 70 % of identity with said fragment 150-313, and
- the fragment 163-313, ,
wherein said polypeptide is fused to a RNA binding protein.

2. The chimeric polypeptide according to claim 1, wherein the sequences having at least 70 % of identity with said fragment 150-313 originate from anyone of the following rotaviruses : bovine rotavirus A, caprine rotavirus A, equine rotavirus A, human rotavirus A, lapine rotavirus, porcine rotavirus A, rabbit rotavirus, rotavirus 5 serotype G2, rotavirus G8, rotavirus subgroup 1, rotavirus subgroup 2, simian rotavirus A/SA11, simian rotavirus A/SA11-C14 and swine rotavirus strain S8.

3. The chimeric polypeptide according to claim 1, whose sequence comprises the sequence SEQ ID N°48.

4. The chimeric polypeptide according to anyone of claims 1 to 3, wherein the RNA binding protein is selected from the group consisting of MS2CP, N, IRP and U1A.

5. The chimeric polypeptide according to anyone of claims 1 to 4, which further comprises a transport signal which allows the chimeric polypeptide to cross the plasma membrane of the target cells.

6. A nucleic acid comprising a polynucleotide encoding a chimeric polypeptide according to anyone of claims 1 to 5.

7. The nucleic acid of claim 6, comprising the polynucleotide of sequence SEQ ID N°49.

8. The nucleic acid according to claim 6 or 7, which further comprises an inducible regulatory polynucleotide for the control of the polynucleotide encoding the chimeric polypeptide according to anyone of claims 1 to 5.

9. A host cell which stably or transiently expresses a chimeric polypeptide according to anyone of claims 1-5, comprising a nucleic acid according to anyone of claims 6 to 8.

10. A protein translation control system of a target polynucleotide of interest comprising a chimeric polypeptide according to anyone of claims 1 to 5, or a nucleic acid according to anyone of claims 6 to 8, or a host cell according to claim 9.

11. Use of a protein translation control system according to claim 10 for activating the translation of a target polynucleotide of interest in a bioreactor comprising host cells in culture.

## Patentansprüche

1. Chimäres Polypeptid, das imstande ist, speziell die Translation eines interessierenden Zielnukleotids zu aktivieren, welches ein Polypeptid umfasst, das aus einem Aminosäurefragment des NSP3-Proteins der Sequenz SEQ ID N°46 besteht, wobei das Aminosäurefragment ausgewählt ist aus der Gruppe bestehend aus:
- dem Fragment 150-313, welches die Sequenz SEQ ID N°35 aufweist,
- irgendeiner Sequenz, die eine Identität von wenigstens 70 % mit dem Fragment 150-313 aufweist, und
- dem Fragment 163-313,
wobei das Polypeptid mit einem RNA-bindenden Protein fusioniert ist.

2. Chimäres Polypeptid gemäß Anspruch 1, wobei die Sequenzen, die eine Identität von wenigstens 70 % mit dem Fragment 150-313 aufweisen, von irgendeinem der folgenden Rotaviren stammen: Bovines Rotavirus A, caprines Rotavirus A, equines Rotavirus A, humanes Rotavirus A, lapines Rotavirus A, porcines Rotavirus A, Kaninchen-Rotavirus, Rotavirus 5 Serotyp G2, Rotavirus G8, Rotavirus Untergruppe 1, Rotavirus Untergruppe 2, Simian-Rotavirus A/SA11, Simian-Rotavirus A/SA11-C14 und Schweine-Rotavirus Stamm S8.

3. Chimäres Polypeptid gemäß Anspruch 1, dessen Sequenz die SEQ ID N°48 umfasst.

4. Chimäres Polypeptid gemäß irgendeinem der Ansprüche 1 bis 3, wobei das RNA-bindende Protein aus der Gruppe bestehend aus MS2CP, N, IRP und U1A ausgewählt ist.

5. Chimäres Polypeptid gemäß irgendeinem der Ansprüche 1 bis 4, welches ferner ein Transportsignal umfasst, das dem chimären Polypeptid einen Durchtritt durch die Plasmamembran der Zielzellen ermöglicht.

6. Nukleinsäure, umfassend ein Polynukleotid, welches ein chimäres Polypeptid gemäß irgendeinem der Ansprüche 1 bis 5 kodiert.

7. Nukleinsäure gemäß Anspruch 6, welche das Polynukleotid der Sequenz SEQ ID N°49 umfasst.

8. Nukleinsäure gemäß Anspruch 6 oder 7, welche ferner ein induzierbares regulatorisches Polynukleotid zur Kontrolle des Polynukleotids, welches das chimäre Polypeptid gemäß irgendeinem der Ansprüche 1 bis 5 kodiert, umfasst.

9. Wirtszelle, welche stabil oder transient ein chimäres Polypeptid gemäß irgendeinem der Ansprüche 1-5 exprimiert, umfassend eine Nukleinsäure gemäß irgendeinem der Ansprüche 6 bis 8.

10. Proteintranslationskontrollsystem eines interessierenden Zielpolynukleotids, umfassend ein chimäres Polypeptid gemäß irgendeinem der Ansprüche 1 bis 5 oder eine Nukleinsäure gemäß irgendeinem der Ansprüche 6 bis 8 oder eine Wirtszelle gemäß Anspruch 9.

11. Verwendung eines Proteintranslationskontrollsystems gemäß Anspruch 10 zur Aktivierung der Translation eines interessierenden Zielpolynukleotids in einem Bioreaktor, welcher die Wirtszellen in Kultur umfasst.

## Revendications

1. Polypeptide chimérique capable d'activer spécifiquement la traduction d'un polynucléotide cible d'intérêt, qui comprend un polypeptide consistant en un fragment d'acides aminés de la protéine NSP3 de séquence SÉQ ID N° 46, ledit fragment d'acides aminés étant choisi dans le groupe consistant en :
- le fragment 150 à 313 qui a la séquence SÉQ ID N° 35,
- toute séquence présentant au moins 70 % d'identité avec ledit fragment 150 à 313, et
- le fragment 163 à 313,
où ledit polypeptide est fusionné à une protéine de liaison d'ARN.

2. Polypeptide chimérique selon la revendication 1, où les séquences présentant au moins 70 % d'identité avec ledit fragment 150 à 313 proviennent de l'un quelconque des rotavirus suivants : rotavirus A bovin, rotavirus A caprin, rotavirus A équin, rotavirus A humain, rotavirus de lapine, rotavirus A porcin, rotavirus de lapin, rotavirus 5 sérotype G2, rotavirus G8, rotavirus sous-groupe 1, rotavirus sous-groupe 2, rotavirus A/SA11 simien, rotavirus A/SA11-C14 simien et souche S8 du rotavirus du porc.

3. Polypeptide chimérique selon la revendication 1, dont la séquence comprend la séquence SÉQ ID N° 48.

4. Polypeptide chimérique selon l'une quelconque des revendications 1 à 3, où la protéine de liaison d'ARN est choisie dans le groupe consistant en MS2CP, N, IRP et U1A.

5. Polypeptide chimérique selon l'une quelconque des revendications 1 à 4, qui comprend en outre un signal de transport qui permet au polypeptide chimérique de traverser la membrane plasmatique des cellules cibles.

6. Acide nucléique comprenant un polynucléotide codant pour un polypeptide chimérique selon l'une quelconque des revendications 1 à 5.

7. Acide nucléique selon la revendication 6, comprenant le polynucléotide de séquence SÉQ ID N° 49.

8. Acide nucléique selon la revendication 6 ou 7, qui comprend en outre un polynucléotide régulateur inductible pour le contrôle du polynucléotide codant pour le polypeptide chimérique selon l'une quelconque des revendications 1 à 5.

9. Cellule hôte qui exprime de façon stable ou transitoire un polypeptide chimérique selon l'une quelconque des revendications 1 à 5, comprenant un acide nucléique selon l'une quelconque des revendications 6 à 8.

10. Système de contrôle de la traduction de protéines d'un polynucléotide cible d'intérêt comprenant un polypeptide chimérique selon l'une quelconque des revendications 1 à 5, ou un acide nucléique selon l'une quelconque des revendications 6 à 8, ou une cellule hôte selon la revendication 9.

11. Utilisation d'un système de contrôle de la traduction de protéines selon la revendication 10 pour l'activation de la traduction d'un polynucléotide cible d'intérêt dans un bioréacteur comprenant des cellules hôtes en culture.
